# EUROPEAN PATENT SPECIFICATION

(11) **EP 4 165 027 B1**
(45) Date of publication and mention of the grant of the patent: **12.02.2025**
(21) Application number: 21733395.4
(22) Date of filing: 10.06.2021
(51) Int. Cl.: C07D 257/02, A61K 49/10, A61K 33/244, A61K 31/555

(54) **PROCEDURE FOR OBTAINING GADOTERATE MEGLUMINE FROM HIGH-PURITY TETRAXETAN (DOTA) AND ITS USE IN THE PREPARATION OF INJECTABLE GALENICAL FORMULATIONS**
VERFAHREN ZUR GEWINNUNG VON GADOTERAT-MEGLUMIN AUS HOCHREINEM TETRAXETAN (DOTA) UND SEINE VERWENDUNG BEI DER HERSTELLUNG VON INJIZIERBAREN GALENISCHEN FORMULIERUNGEN
PROCÉDURE D'OBTENTION DE GADOTÉRATE MÉGLUMINE À PARTIR DE TÉTRAXÉTAN (DOTA) DE HAUTE PURETÉ ET SON UTILISATION DANS LA PRÉPARATION DE FORMULATIONS GALÉNIQUES INJECTABLES

(30) Priority: 10.06.2020 EP 20382500
(43) Date of publication of application: 19.04.2023
(73) Proprietor: Justesa Imagen S.A.U., 28823 Coslada (ES)
(72) Inventor: ALONSO SILVA, Ignacio, 28823 Coslada (ES); CERECEDA PÉREZ, Juan Andrés, 28823 Coslada (ES); MARTÍN MARTÍN, Felix Ramón, 28823 Coslada (ES); REGUEIRA GÓMEZ, Maria Ángeles, 28823 Coslada (ES); CHICHARRO MARTÍN, Roberto, 28823 Coslada (ES); GARCÍA RAMOS, Álvaro, 28823 Coslada (ES); GARCÍA SALADO, Irene, 28223 Coslada (ES); MONTIEL LEGUEY, Vicente, 03690 San Vicente del Raspeig (Alicante) (ES); EXPÓSITO RODRÍGUEZ, Eduardo, 03690 San Vicente del Raspeig (Alicante) (ES); GARCÍA GARCÍA, Vicente, 03690 San Vicente del Raspeig (Alicante) (ES); VALERO VALERO, David, 03690 San Vicente del Raspeig (Alicante) (ES); SÁEZ FÉRNANDEZ, Alfonso, 03690 San Vicente del Raspeig (Alicante) (ES); GALLUD MARTINÉZ, Francisco, 03690 San Vicente del Raspeig (Alicante) (ES)
(74) Representative: Elzaburu S.L.P.
(86) International application number: PCT/EP2021/065599
(87) International publication number: WO 2021/250163

(56) References cited:
- EP-A1- 3 564 218
- WO-A1-2015/117911
- WO-A1-2017/103258
- CN-A- 108 658 882
- FENGCHENG WU ET AL: "Novel polyazamacrocyclic receptor decorated core-shell superparamagnetic microspheres for selective binding and magnetic enrichment of palladium: synthesis, adsorptive behavior and coordination mechanism", DALTON TRANSACTIONS, vol. 45, no. 23, 1 January 2016 (2016-01-01), pages 9553 - 9564, XP055637564, ISSN: 1477-9226, DOI: 10.1039/C6DT01024E

## Description

### FIELD OF THE INVENTION

The present invention relates to a process for obtaining gadoterate meglumine from high purity tetraxetan which does not require the use of organic solvents and optimizes the conditions of the synthesis. Gadoterate meglumine is used as a contrast agent in diagnostic tests. Therefore, the present invention can be included in the field of pharmacology or pharmaceutical chemistry.

### BACKGROUND OF THE INVENTION

Diagnostic imaging is a technique widely used in the field of medicine for the visualization of biological processes, organs or tissues, which requires the use of contrast media. One type of these contrast media is gadolinium-based derivatives, which are compounds increasingly used in magnetic resonance imaging (MRI) studies. However, the toxicity of linear gadolinium-based contrast agents and the existence of free gadolinium deposits in certain areas of the brain have caused that already existing, but not widely used, macrocyclic derivatives arise as a safer alternative and with less accumulation of free gadolinium. Among the macrocyclic derivatives, gadoterate meglumine is the compound with the least accumulation of gadolinium in the brain, possibly due to its high stability (Am. J. Neuroradiol. 2016, 37, 1192-1198).

The synthetic process to obtain tetraxetan, a precursor of gadoterate meglumine, involves tedious and expensive purification processes that, in most cases, use, among others, ion exchange resins that require subsequent treatment with organic solvents to desorb the compound of interest. US5922862 describes the purification of tetraxetan and its derivatives by elution through PVP resin. US5334729 discloses the purification of these complexes by using cation exchange columns.

EP3223863 and EP2799090 describe the final formulation of a 0.5 M solution of gadoterate meglumine in water for injections from isolated tetraxetan, gadolinium oxide and meglumine. This process involves successive and long adjustment steps of tetraxetan and gadolinium oxide amounts, so that a tetraxetan content between 0% and 0.25% remains. Then, there is another pH adjustment step with meglumine and finally a concentration adjustment to obtain a 0.5 M solution, which is used as a contrast medium.

WO2017/103258 describes a method for the synthesis of DOTA comprising steps of crystallizing and filtering using membrane filtration, although in both steps methanol is added, remaining a content of methanol of ca. 4% wt. in the final product.

Therefore, there is a need to optimize the tetraxetan synthetic process to reduce the number of steps to obtain high-purity final product. In parallel, it is necessary to minimize the degradation of reagents and intermediates during the process and eliminate the use of organic solvents in order to reduce the environmental impact and possible impurities in the final product.

### DESCRIPTION OF THE INVENTION

The present invention solves the drawbacks of the procedures of the state of the art optimizing the synthetic process to obtain tetraxetan by using milder reaction conditions leading to lower degradation of the starting reagents, and carrying out a combination of purification and isolation techniques which allow obtaining high purity tetraxetan without the use of organic solvents (green chemistry). From this tetraxetan, gadoterate meglumine is obtained, an API which is used with a simple process of dissolution and concentration adjustment in the preparation of aqueous formulations for injections for subsequent use in magnetic resonance imaging diagnosis. The present invention greatly simplifies the process for obtaining gadoterate meglumine by optimizing the manufacturing process and isolating the gadoterate meglumine as a solid which meets well-defined specifications and that, due to the use of high purity tetraxetan, does not require any additional purification process.

Therefore, in a first aspect the present invention refers to a procedure to obtain tetraxetan comprising the following steps:
(a) obtaining of tetraxetan by the reaction described below: 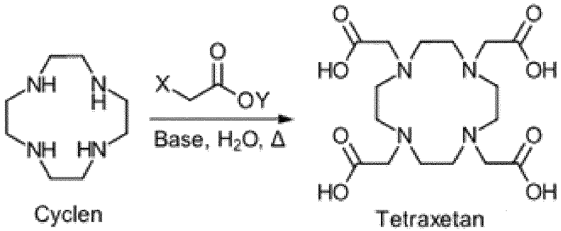 where X is a halogen, preferably chlorine, Y is selected from hydrogen or an alkaline element, preferably sodium, the base is selected from potassium, sodium or lithium hydroxide, preferably sodium hydroxide, the pH is maintained between 7 and 8.5, preferably 8 and the reaction temperature is kept between 70 and 100°C, preferably 80°C, and crystallization of the obtained tetraxetan by lowering the pH below 3 to obtain a tetraxetan crude;
(b) purification of the crystallized tetraxetan crude obtained in the previous step, involving at least one electrodialysis;
(c) isolation of the product obtained in the previous step, preferably by spray drying.

In the present invention, tetraxetan is also referred as DOTA.

In a preferred embodiment, the crystallization of the tetraxetan obtained in the reaction of step (a) is carried out at a pH below 3, preferably equal or below 2 and more preferably equal or below 1. This decrease of pH may be carried out by adding an acid commonly used, such as HCl. These pH values allow a good yield since they favour the transformation of all carboxylate groups to carboxylic acids and avoid the use of organic solvents as no purification is necessary. Variations of pH of ± 0.2 to ±0.5 are considered within the scope of the invention and may be applied to the described pH values.

In a preferred embodiment, cationic, anionic, bipolar membranes or combinations thereof are used in electrodialysis.

In another preferred embodiment, two consecutive electrodialysis are performed in the purification step (b). In a first more preferred embodiment, the first electrodialysis is performed using:
- a combination of cationic, anionic and bipolar membranes or
- a combination of cationic and anionic membranes where preferably the anionic membranes are monoselective.

In another, more preferred procedure, the second electrodialysis is carried out using:
- a combination of cationic, anionic and bipolar membranes or
- a combination of cationic and bipolar membranes.

Preferably, in both electrodialysis of this preferred embodiment, the pH is maintained between 2 and 6.

In another preferred embodiment, in the purification step (b), a nanofiltration with constant volume is performed prior to electrodialysis. In a more preferred embodiment, the electrodialysis is performed using:
- a combination of cationic, anionic and bipolar membranes or
- a combination of cationic and anionic membranes where preferably the anionic membranes are monoselective or
- a combination of cationic and bipolar membranes.

Preferably, the pH during nanofiltration is maintained between 2 and 8, more preferably between 3 and 5 and even more preferably at 4.

Preferably, the pH during electrodialysis of this preferred embodiment is kept between 2 and 5, more preferably at 4.

In a preferred embodiment, in step (c) the compound is isolated by spray drying in which the temperature of the inlet air is 160 - 200ºC. In a more preferred embodiment, the inlet air temperature is 170 - 190ºC. In a more preferred embodiment, the inlet air temperature is 175 - 185ºC. In an even more preferred embodiment, the inlet air temperature is 180°C. In a preferred embodiment, the outlet air temperature is 90 - 120°C. In a more preferred embodiment, the outlet air temperature is 105 - 115ºC. In an even more preferred embodiment, the outlet air temperature is 110°C.

The spray-drying step involves a fundamental improvement of the process of the invention in respect to other similar process known in the art. Due to the high-purity of DOTA obtained in step (b) there is no need to use organic solvents to isolate the product as described in, for example, WO2017/103258 where high amounts of methanol are used in several steps, and the final product presented traces of said methanol. Therefore, by applying the process of the present invention the final product is free of organic solvents, something that is advantageous for the manufacturing of a pharmaceutical product.

In another preferred embodiment, the product obtained in step (c) is characterised by having a maximum residual amount of the alkaline element, preferably sodium, of 500ppm (0.05%) and a maximum residual amount of halide, preferably chloride, of 500ppm (0.05%).

The present application also describes a tetraxetan obtained by the process described above, characterised by having (a) a maximum residual amount of the alkaline element, preferably sodium, determined for example by inductively coupled plasma mass spectrometry (ICP-MS), of 500ppm (0.05%), preferably 100ppm (0.01%), more preferably 50ppm (0.005%), (b) a maximum residual amount of halide, preferably chloride, determined, for instance, by inductively coupled plasma mass spectrometry (ICP-MS), of 500ppm (0.05%), preferably 100ppm (0.01%), more preferably 50ppm (0.005%), even more preferably 20ppm (0.002%), and (c) a maximum residual amount of solvents and volatile substances below the detection limit.

In the present invention, "limit of detection" (LOD) or "detection limit" is defined as the lowest quantity of an analyte whose signal can be distinguished from the absence of that substance ("noise") with a stated confidence level and is usually defined as the minimum amount or concentration of substance that can be reliably detected by a given analytical method. In practice, the limit of detection would be the minimum concentration obtained from the analysis of a sample (containing the analyte) that can be discriminated from the concentration obtained from the determination of a blank sample, *i.e.,* a sample with no analyte present. In this respect, the limit of detection of the possible solvents used in the procedure of this invention can be set at 10ppm (0.001%) determined by means of e.g., gas chromatography/mass spectrometry (GC-MS).

The procedure of the present invention allows obtaining high yields and/or higher levels of purity than the tetraxetan obtained by means of previously described procedures in the state of the art. The purity of the tetraxetan obtained by the process described in the present invention is typically at least over 50%, at least over 60%, at least over 70%, at least over 80%, at least over 90% or more. The purity of the obtained tetraxetan can be measured by methods known to anyone skilled in the art, such as inductively coupled plasma mass spectrometry (ICP-MS), high performance liquid chromatography (HPLC) or gas chromatography/mass spectrometry (GC-MS), as will be shown in the examples.

The term "halogen" relates to chlorine (CI), bromine (Br) and iodine (I). In their anionic form, they are mentioned as halides.

The term "alkaline" means lithium (Li), sodium (Na) and potassium (K).

The term "base" means a substance which when dissolved in an aqueous medium releases hydroxyl ions (OH⁻) and gives to the medium alkaline properties. Preferably, it refers to alkaline hydroxides such as potassium hydroxide (KOH), sodium hydroxide (NaOH) and lithium hydroxide (LiOH).

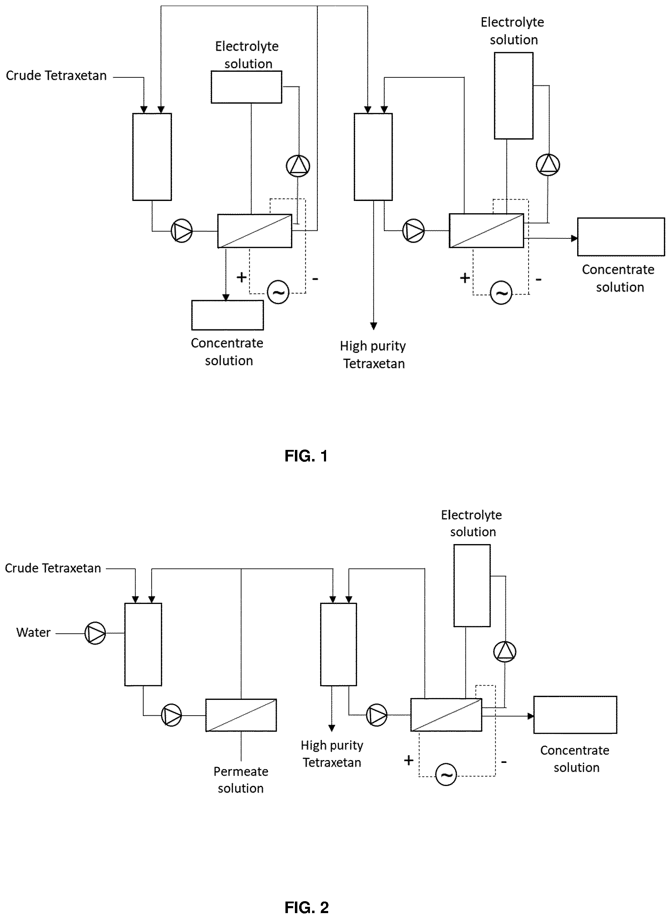

The term "electrodialysis" relates to a membrane process in which ions are transported through an ion exchange membrane by an electrical field as driving force. The electrodialysis system consists of an electrolyte solution, a concentrate solution and a diluate solution. In the present invention, "electrolyte solution" refers to a solution of sulfuric acid at pH=1-3 or to a diluted solution of sodium sulfate. In the present invention, "concentrate solution" refers to a diluted solution of sulfuric acid in water. In the present invention, "diluate solution" refers to a solution of tetraxetan. The electrodialysis procedure of the invention can be carried out with different configurations for industrial scale-up. Nonlimiting examples of possible configurations are shown in Figures 1 and 2.

In the present invention, a control of the base addition is made in order to minimise the formation of salts in the reaction crude. Additionally, the treatment of the reaction crude with electrodialysis and/or nanofiltration allows the removal of inorganic ions in addition to other impurities present in the reaction crude, such as organic impurities.

Another aspect of the invention refers to a process to obtain gadoterate meglumine, which comprises the steps of the process to obtain tetraxetan as described above and the following additional steps:
(d) reaction of the product obtained in step (c) with a gadolinium derivative, preferably Gd₂O₃, and meglumine,
(e) isolation of the product obtained in step (d) by spray drying.

In the present invention, the high quality DOTA obtained in the previous steps (a)-(c) allows directly isolating the reaction mass of step (d) on solution by spray drying step without any purification step required. This fact increases yields and eliminates the need to use organic solvents, compared to other techniques of the art in which meglumine gadoterate is isolated from the solution a slurry by crystallization with organic solvents.

To a tetraxetan solution, previously purified by any of the above-described techniques, the corresponding gadolinium derivative (1 eq.) is added. The mixture is heated for a certain time. The pH of the reaction is kept constant during the whole process by adding of meglumine together with the gadolinium derivative in step (d). This improves the reaction times since it generates meglumine gadoterate directly and prevents the formation of highly water insoluble gadolinium hydroxides.

In a preferred embodiment, the initial tetraxetan concentration is 50-250g/L.

In the present invention, "gadolinium derivative" refers to gadolinium oxide or a gadolinium salt, such as gadolinium chloride, gadolinium sulfate, etc. Preferably, the gadolinium derivative is gadolinium oxide.

In a preferred embodiment, the pH of the reaction remains constant. In a preferred embodiment, the pH of the reaction is between pH = 1-7. In a more preferred embodiment, the pH of the reaction is between pH =2-5. In an even more preferred embodiment, the pH of the reaction is between pH = 3.5-4.5. In a preferred embodiment, the pH of the reaction is maintained by addition of meglumine.

In a preferred embodiment, the solvent is water.

In a preferred embodiment, the temperature of the reaction is between 80-100ºC. In a more preferred embodiment, the temperature of the reaction is 85°C.

In a preferred embodiment, the final pH of the solution is adjusted to a pH value that is between 6.5-8 by adding meglumine. In another preferred embodiment, the final pH of the solution is adjusted to a pH value that is between 6.5 and 7.5 by the addition of meglumine. In a preferred embodiment, in step (e) the compound is isolated by spray drying. During the process the temperature of the inlet air is between 160-200ºC. In a more preferred embodiment, the inlet air temperature is between 170-190ºC. In a more preferred embodiment, the inlet air temperature is between 175-185ºC. In an even more preferred embodiment, the inlet air temperature is 180°C. In another preferred embodiment, the outlet air temperature is between 90-120ºC. In a more preferred embodiment, the outlet air temperature is between 105-115°C. In an even more preferred embodiment, the outlet air temperature is 110°C.

In a preferred embodiment, the product obtained in step (d) is subjected to at least one ultrafiltration.

The gadoterate meglumine obtained in this way does not require any further purification process, so it can be directly used to prepare pharmaceutical compositions.

The present application describes a gadoterate meglumine obtained by the process according to the claim characterized by a maximum amount of residual solvents and other volatile substances below the detection limit. The detection limit of the solvents (which may be, for example, those mentioned above) is approximately 10ppm (0.001%). For other related substances such as cyclen, chloroacetic acid, glycolic acid, etc. it is approximately 0.001%, determined by e.g. high performance liquid chromatography (HPLC).

The present application also describes a pharmaceutical composition comprising gadoterate meglumine as described above, preferably such a pharmaceutical composition is a contrast agent formulated as injectable.

To obtain this pharmaceutical composition, procedures and techniques known to anyone skilled in the art and well established by the applicable pharmacopoeia shall be used. For example, in the case of injectables, the gadoterate meglumine will be dissolved in water for injection according to the desired concentration and the corresponding treatment will be carried out to finally obtain the vials ready for use.

### BRIEF DESCRIPTION OF THE FIGURES

Figure 1.- Shows the scheme of the tetraxetan purification process by means of two electrodialysis.
Figure 2.- Shows the scheme of the tetraxetan purification process by means of nanofiltration followed by electrodialysis.

### EXAMPLES OF THE INVENTION

### Example 1. Synthesis of raw tetraxetan

A 125 g/L solution of cyclen (1 eq.) in water is prepared. Then chloroacetic acid (4.5 eq.) is added. The mixture is heated to 80ºC and the pH of the reaction is adjusted to 8, adding sodium hydroxide using a pH controller. When the reaction is completed, the pH is raised to 10 maintaining the temperature for the necessary time. The reaction mixture is then cooled to 65°C and then concentrated HCl is added until pH<1. Finally, the solvent is partially removed under reduced pressure, the mixture is cooled and the tetraxetan crude obtained is centrifuged (80-85% yield).

### Example 2. Tetraxetan purification by a combination of nanofiltration and/or electrodialysis techniques

### A. Treatment of raw tetraxetan by means of two electrodialysis:

A solution of raw tetraxetan (45g/L) is purified by a first electrodialysis containing monoselective anionic membranes and cationic membranes. The pH of the diluate solution is maintained between 2-5. The concentrate and electrolyte solutions can be sulfuric acid at pH=1-3 or sodium sulfate at a concentration of 5g/L (95% yield; Cl<0.01%; Na<0.5%).

This electrodialysed solution is then purified by a second electrodialysis containing cationic and bipolar membranes. The pH of the diluate solution is maintained between pH=2.8-4.5. The concentrate and electrolyte solutions are as described above. In this way, a tetraxetan solution is obtained which is directly used in the next isolation step (90-95% yield; Na<0.01%).

### B. Treatment of raw tetraxetan by means of nanofiltration and electrodialysis:

A 45g/L solution of raw tetraxetan is prepared and the pH is adjusted to 4 by adding sodium hydroxide solution. This solution is passed through the nanofiltration membrane, resulting in two streams, the rejection solution, which returns to the initial solution containing tetraxetan, and the permeate solution, which is collected in a different tank. This last solution contains inorganic ions and low molecular weight organic impurities. The volume of the rejected solution is kept constant by the addition of water. The nanofiltration process ends when the concentration of chloride anions is below the value of the specification (98-99% yield; Cl<0.01%; Na<4.1%)

This tetraxetan solution at a concentration of 45g/L is then subjected to an electrodialysis process in which cationic and anionic membranes are used, and the pH of the diluate solution is maintained between pH=2.8-4.5. Or, a tetraxetan solution is treated with cationic membranes and monoselective anionic membranes, and the pH of the diluate solution is maintained between pH=2-3. Or, a tetraxetan solution at a concentration of 45g/L is treated with cationic and bipolar membranes, and the pH of the diluate solution is maintained at pH=4. The electrolyte and concentrate solutions are a sulfuric acid or sodium sulfate solution. The electrodialysis process ends when the concentration of sodium cations is less than the specification value. In this way, a tetraxetan solution is obtained which goes directly to the isolation step (90-95% yield; Na<0.008%).

### Example 4. Isolation and characterization of tetraxetan

The solution from the purification process can be used in the synthesis of gadoterate meglumine or, alternatively, tetraxetan can be isolated. Tetraxetan can be isolated from the aqueous solution by spray drying at an inlet air temperature of 180°C and an outlet air temperature of 110°C. The result is a product that meets the specifications described below (97-99% yield; Na<0.008%).

The high purity tetraxetan obtained meets the following specifications:
- Appearance: white powder
- Identification:
   ∘ IR spectrum: comparable with the reference tetraxetan
   ∘ HPLC: retention time matching that of the reference
- Water ≤ 8.5%
- Clear solution, no more coloured than reference solution Y7
- Sodium ≤ 500ppm (0.05%)
- Halides ≤ 500ppm (0.05%)
- Residue on ignition ≤ 0.10%
- Related impurities:
   ∘ DO3A ≤ 0.05%
   ∘ Any other individual impurity ≤ 0.05%
   ∘ Total impurities ≤ 0.5%
- Titration (acid-base): 98.0-102.0% on dry product
- Bacterial Endotoxin ≤ 22 EU/g
- Total aerobic microbial count ≤ 10² CFU/g
- Total combined yeast and mould count ≤ 10² CFU/g
- *Escherichia coli*: absence/1g
- Residual solvents < 10ppm (0.001%)

### Example 5. Gadoterate meglumine synthesis

To a solution of tetraxetan in water (200g/L), Gd₂O₃ (1 eq.) is added. The mixture is heated to 85ºC for 2-4 h. The pH of the reaction is maintained at pH=4 by adding meglumine. The reaction ends when the final amount of tetraxetan and free gadolinium is less than 0.005% (w/v). The pH of the solution is then raised between pH=6.5-8 by the addition of meglumine (quantitative yield)

### Example 6. Isolation and characterization of gadoterate meglumine

The final product dissolved in water is subjected to depyrogenizing ultrafiltration and then isolated by spray drying. The working conditions are as follows, inlet air temperature between 175-185ºC and outlet air temperature 110ºC (97-98% yield). The gadoterate meglumine isolated by this procedure has the following specifications:
- Appearance: white powder
- Water ≤ 10.0%
- Identification:
   ∘ HPLC: retention time similar to that of the reference
   ∘ IR spectrum: similar to the reference
- Clear or no more opalescent solution than European Pharmacopoeia and American Pharmacopoeia (USP) reference solution I for the clarity and opalescence test of solutions
- Colour of the solution ≤ Y7
- pH=6.5-8
- Related impurities:
   ∘ DO3A ≤ 0.05%
   ∘ Tetraxetan ≤ 0.1%
   ∘ Any other individual impurity ≤ 0.05%
   ∘ Total impurities ≤ 0.50%
- Purity (HPLC): 97-103% on anhydrous product
- Meglumine content: 24.5-26.5% on anhydrous product
- Free Gd content ≤ 0.01%
- Total Gd content: 19-22%.
- Foreign matter ≤ 100ppm (0.01%)
- Bacterial endotoxin < 20UE/g
- Total aerobic microbial count (TAMC) ≤ 10² CFU/g
- Total combined yeast and mould count (TYMC) ≤ 10² CFU/g
- *Escherichia coli*: absence
- Residual solvents <10ppm (0.001%)
- Elemental impurities:
   ∘ As ≤1500ppb
   ∘ Pb ≤500ppb
   ∘ Cd ≤200ppb
   ∘ Hg ≤300ppb
   ∘ V ≤1000ppb
   ∘ Ni ≤2000ppb
   ∘ Co ≤500ppb
   ∘ Pd ≤1000ppb
   ∘ Cu ≤30000ppb
   ∘ Li ≤25000ppb
   ∘ Sb ≤9000ppb

### Example 7. Galenic formulation of gadoterate meglumine. (not according to the invention).

With the obtained gadoterate meglumine, injectable galenic formulations were prepared to be used in magnetic resonance imaging diagnosis.

**Table 1. Solution of gadoterate meglumine in water at a concentration of 0.5 M.**

| | | |
|---|---|---|
| Gadoterate meglumine | 1g | 0.5M |
| WFI* up to a total volume of | 2.65mL | |

| | | |
|---|---|---|
| *WFI, water for injection. | | |

**Table 2. Solution of 0.5M gadoterate meglumine and tetraxetan as excipient in water.**

| | | |
|---|---|---|
| Gadoterate meglumine | 1g | 0.5M |
| WFI up to a volume of | 2.65mL | |
| Tetraxetan (excipient) | 0.133-1.33mg | 0.025-0.25% (mol/mol) |

These two prepared formulations with the gadoterate meglumine isolated from the present invention are depyrogenized and sterilized. These two formulations meet the following specifications:
- Clear solution, no more coloured than the reference solution Y7 of the European pharmacopoeia and the American pharmacopoeia (USP) for the test of clarity and opalescence of solutions
- Identification:
   ∘ IR spectrum: similar to the reference compound
   ∘ HPLC: retention time similar to the reference standard.
- pH=6.9-8.
- Density = 1.1649-1.1828g/mL
- Free of visible particles.
- Subvisible particles < 6000 (particle size < 10µm); < 600 (particle size < 25µm)
- Absorbance: at 450nm < 0.40AU; at 500nm < 0.20AU
- Assay:
   ∘ Gadoterate: 26.53 - 29.33% (w/v)
   ∘ Total gadolinium: 7.63 - 8.10% (w/v)
   ∘ Free gadolinium < 0.005% (w/v)
   ∘ Meglumine: 9.27 - 10.25% (w/v)
- Related impurities:
   ∘ DO3A < 0.02% (w/v)
   ∘ Tetraxetan < 0.05% (w/v)
   ∘ Any other impurity < 0.02% (w/v)
   ∘ Total impurities < 0.2% (w/v)
- Elemental impurities:
   ∘ As ≤ 560ppb
   ∘ Pb ≤ 190ppb
   ∘ Cd ≤ 75ppb
   ∘ Hg ≤ 110ppb
   ∘ V ≤ 370ppb
   ∘ Ni ≤ 750ppb
   ∘ Co ≤ 190ppb
   ∘ Pd ≤ 370ppb
   ∘ Cu ≤ 1100ppb
   ∘ Li ≤ 940ppb
   ∘ Sb ≤ 3400ppb
- Sterile solution, free of bacterial endotoxins (< 8EU/mL).

### Example 8. Comparison of tetraxetan obtained by the process of the invention.

A comparison between the levels of present impurities in tetraxetan samples obtained by the process of the invention (DOTA 001-003) and other commercially available tetraxetan samples from different suppliers (Supplier 1-5) was made. The data are summarized in the following tables:

**Table 3. Determination of elemental alkaline and halide impurities in tetraxetan by inductively coupled plasma mass spectrometry (ICP-MS)**

| **SAMPLE** | **Na (ppm) (LOD 1ppm)** | **K (ppm) (LOD 1ppm)** | **Cl (ppm) (LOD 2ppm)** | **Br (ppm) (LOD 10ppm)** |
|---|---|---|---|---|
| Supplier 1 | 62 | 7 | 60 | n.d. |
| Supplier 2 | 68 | n.d. | 68 | n.d. |
| Supplier 3 | 82 | n.d. | 121 | n.d. |
| Supplier 4 | 20550 | 500 | 8500 | 49000 |
| Supplier 5 | 1500 | 150 | 50 | n.d. |
| DOTA-001 | 45 | n.d. | 2 | n.d. |
| DOTA-002 | 59 | n.d. | 12 | n.d. |
| DOTA-003 | 47 | n.d. | 13 | n.d. |

**Table 4. Determination of residual solvents in tetraxetan by gas chromatography/mass spectrometry (GC-MS)**

| **SAMPLE** | **Ethanol (ppm) (LOD10 ppm)** | **Ethyl acetate (ppm) (LOD10 ppm)** | **Acetone (ppm) (LOD10 ppm)** | **Ammonia (ppm) (LOD10 ppm)** | **Methanol (ppm) (LOD10 ppm)** | **Isopropanol (ppm) (LOD10 ppm)** |
|---|---|---|---|---|---|---|
| Supplier 1 | 395 | n.d. | 20 | | | n.d. |
| Supplier 2 | n.d. | 2 | 44 | | | n.d. |
| Supplier 3 | n.d. | n.d. | 10 | | 25 | n.d. |
| Supplier 4 | 75 | n.d. | n.d. | | | n.d. |
| Supplier 5 | 1500 | n.d. | n.d. | 67000 | | 160 |
| DOTA-001 | n.d. | n.d. | n.d. | n.d. | n.d. | n.d. |
| DOTA-002 | n.d. | n.d. | n.d. | n.d. | n.d. | n.d. |
| DOTA-003 | n.d. | n.d. | n.d. | n.d. | n.d. | n.d. |

**Table 5. Tetraxetan purity determined by HPLC for each sample**

| **SAMPLE** | **DOTA (%)** |
|---|---|
| Supplier 1 | 99.88 |
| Supplier 2 | 99.85 |
| Supplier 3 | 99.86 |
| Supplier 4 | 90.81 |
| Supplier 5 | 96.30 |
| DOTA-001 | 99.99 |
| DOTA-002 | 99.99 |
| DOTA-003 | 99.99 |

**Table 6. Determination of other impurities by high performance liquid chromatography (HPLC)**

| **SAMPLE** | **CYCLEN (%) (LOD 0.001%)** | **DO3A (%) (LOD 0.001%)** | **Chloroacetic acid (%) (LOD 0.001%)** | **Glycolic acid (%) (LOD 0.001%)** | **Any other individual impurity (%) (LOD 0.001%)** | **Total impurities (%) (LOD 0.001%)** |
|---|---|---|---|---|---|---|
| Supplier 1 | 0.006 | n.d. | 0.083 | 0.005 | 0.035 | 0.117 |
| Supplier 2 | 0.006 | 0.009 | 0.080 | n.d. | 0.057 | 0.152 |
| Supplier 3 | 0.009 | n.d. | 0.084 | 0.011 | 0.016 | 0.100 |
| Supplier 4 | n.d. | 0.003 | n.d. | n.d. | 0.041 | 0.190 |
| Supplier 5 | n.d. | 0.260 | n.d. | n.d. | n.d. | 0.420 |
| DOTA-001 | n.d. | 0.004 | n.d. | n.d. | 0.004 | 0.008 |
| DOTA-002 | n.d. | 0.006 | n.d. | n.d. | 0.003 | 0.009 |
| DOTA-003 | n.d. | n.d. | n.d. | n.d. | 0.009 | 0.009 |

## Claims

1. A process to obtain tetraxetan that comprises the following steps:
(a) obtention of tetraxetan by the following reaction: 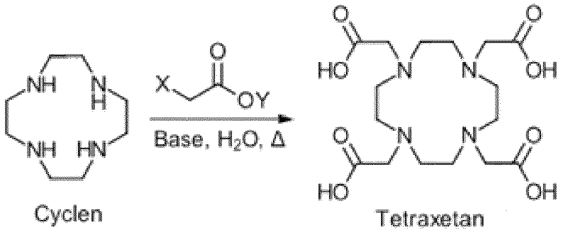 where X is a halogen, Y is selected from hydrogen or an alkaline element, the base is selected from potassium, sodium or lithium hydroxide, the pH is maintained between 7 and 8.5 and the reaction temperature is maintained between 70 and 100°C, and crystallization of the obtained tetraxetan by lowering the pH below 3 to obtain a tetraxetan crude;
(b) purification of the crystallized tetraxetan crude obtained in the previous step, involving at least one electrodialysis;
(c) isolation of the product obtained in the previous step by spray drying.

2. The process according to claim 1, where X is chlorine and/or Y is sodium.

3. The process according to any one of the claims 1 or 2, where the base is sodium hydroxide.

4. The process according to any one of the claims 1 to 3, where the pH of the reaction is maintained at 8 and/or the temperature is maintained at 80ºC.

5. The process according to any one of the claims 1 to 4, where the pH in the crystallization of step (a) is below 2, preferably below 1.

6. The process according to any one of claims 1 to 5, where cationic, anionic, bipolar membranes or combinations thereof are used in electrodialysis.

7. The process according to any of the claims 1 to 6, where in the step (b) of purification two consecutive electrodialysis are performed.

8. The process according to claim 7, where the first of the two electrodialysis is carried out using:
- a combination of cationic, anionic and bipolar membranes or
- a combination of cationic and anionic membranes, where preferably the anionic membranes are monoselective.

9. The process according to any of the claims 7 or 8, where the second electrodialysis is carried out using:
- a combination of cationic, anionic and bipolar membranes or
- a combination of cationic and bipolar membranes.

10. The process according to any of the claims 7 to 9, where in both electrodialysis the pH is maintained between 2 and 6.

11. The process according to any of the claims 1 to 6, where in the step (b) of purification a nanofiltration at constant volume is performed prior to electrodialysis.

12. The process according to claim 11, where electrodialysis is carried out using:
- a combination of cationic, anionic and bipolar membranes or
- a combination of cationic and anionic membranes, where preferably the anionic membranes are monoselective or
- a combination of cationic and bipolar membranes.

13. The process according to any of the claims 11 or 12, where the pH during nanofiltration is kept between 2 and 8, preferably between 3 and 5, more preferably at 4.

14. The process according to any of the claims 11 to 13, where the pH during electrodialysis is kept between 2 and 5, preferably at 4.

15. A process to obtain gadoterate meglumine comprising the steps of the process according to claims 1 to 14 and the following additional steps:
(d) reaction of the product obtained in step (c) with Gd₂O₃ and meglumine
(e) isolation of the product obtained in step (d) by spray drying, preferably wherein the temperature of the inlet air to the spray drying system is 160-200°C, preferably 170-190°C, more preferably 175-185°C, even more preferably 180°C and/or the temperature of the outlet air is 90-120°C, preferably 105-115°C, more preferably 110°C.

## Patentansprüche

1. Verfahren zur Gewinnung von Tetraxetan, das die folgenden Schritte umfasst:
(a) Gewinnung von Tetraxetan durch folgende Reaktion: 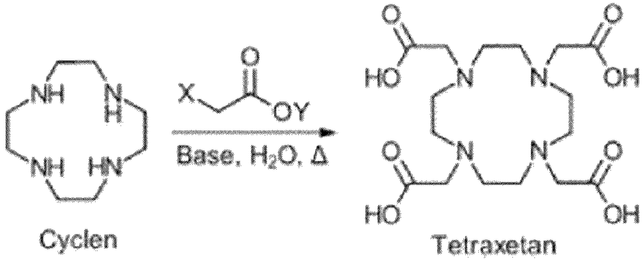 wobei X ein Halogen ist, Y ausgewählt ist aus Wasserstoff oder einem alkalischen Element, die Base ausgewählt ist aus Kalium-, Natrium- oder Lithiumhydroxid, der pH-Wert zwischen 7 und 8,5 und die Reaktionstemperatur zwischen 70 und 100ºC gehalten wird, und Kristallisation des erhaltenen Tetraxetans durch Absenken des pH-Wertes unter 3, um ein Tetraxetan-Rohprodukt zu erhalten;
(b) Reinigung des im vorherigen Schritt erhaltenen kristallisierten Tetraxetan-Rohprodukts unter Einbeziehung mindestens einer Elektrodialyse;
(c) Isolierung des im vorherigen Schritt erhaltenen Produkts durch Sprühtrocknung.

2. Verfahren nach Anspruch 1, wobei X Chlor und/oder Y Natrium ist.

3. Verfahren nach einem der Ansprüche 1 oder 2, wobei die Base Natriumhydroxid ist.

4. Verfahren nach einem der Ansprüche 1 bis 3, wobei der pH-Wert der Reaktion bei 8 und/oder die Temperatur bei 80ºC gehalten wird.

5. Verfahren nach einem der Ansprüche 1 bis 4, wobei der pH-Wert bei der Kristallisation von Schritt (a) unter 2, vorzugsweise unter 1, liegt.

6. Verfahren nach einem der Ansprüche 1 bis 5, wobei kationische, anionische, bipolare Membrane oder Kombinationen davon in der Elektrodialyse verwendet werden.

7. Verfahren nach einem der Ansprüche 1 bis 6, wobei in Schritt (b) der Reinigung zwei aufeinanderfolgende Elektrodialysen durchgeführt werden.

8. Verfahren nach Anspruch 7, wobei die erste der beiden Elektrodialysen durchgeführt wird, unter Verwendung von:
- einer Kombination aus kationischen, anionischen und bipolaren Membranen oder
- einer Kombination aus kationischen und anionischen Membranen, wobei vorzugsweise die anionischen Membranen monoselektiv sind.

9. Verfahren nach einem der Ansprüche 7 oder 8, wobei die zweite Elektrodialyse durchgeführt wird, unter Verwendung von:
- einer Kombination aus kationischen, anionischen und bipolaren Membranen oder
- einer Kombination aus kationischen und bipolaren Membranen.

10. Verfahren nach einem der Ansprüche 7 bis 9, wobei bei beiden Elektrodialysen der pH-Wert zwischen 2 und 6 gehalten wird.

11. Verfahren nach einem der Ansprüche 1 bis 6, wobei in Schritt (b) der Reinigung vor der Elektrodialyse eine Nanofiltration bei konstantem Volumen durchgeführt wird.

12. Verfahren nach Anspruch 11, wobei die Elektrodialyse durchgeführt wird, unter Verwendung von:
- einer Kombination aus kationischen, anionischen und bipolaren Membranen oder
- einer Kombination aus kationischen und anionischen Membranen, wobei vorzugsweise die anionischen Membranen monoselektiv oder
- eine Kombination aus kationischen und bipolaren Membranen sind.

13. Verfahren nach einem der Ansprüche 11 oder 12, wobei der pH-Wert während der Nanofiltration zwischen 2 und 8, vorzugsweise zwischen 3 und 5, bevorzugter bei 4 gehalten wird.

14. Verfahren nach einem der Ansprüche 11 bis 13, wobei der pH-Wert während der Elektrodialyse zwischen 2 und 5, vorzugsweise bei 4, gehalten wird.

15. Verfahren zur Gewinnung von Gadoterat-Meglumin, umfassend die Schritte des Verfahrens nach den Ansprüchen 1 bis 14 und die folgenden zusätzlichen Schritte:
(d) Reaktion des in Schritt (c) erhaltenen Produkts mit Gd₂O₃ und Meglumin
(e) Isolierung des in Schritt (d) erhaltenen Produkts durch Sprühtrocknung, wobei die Temperatur der Einlassluft in das Sprühtrocknungssystem vorzugsweise 160-200ºC, vorzugsweise 170-190ºC, bevorzugter 175-185ºC, noch bevorzugter 180ºC beträgt und/oder die Temperatur der Auslassluft 90-120ºC, vorzugsweise 105-115ºC, bevorzugter 110ºC beträgt.

## Revendications

1. Procédé d'obtention de tétraxétan qui comprend les étapes suivantes :
(a) obtention de tétraxétan par la réaction suivante : 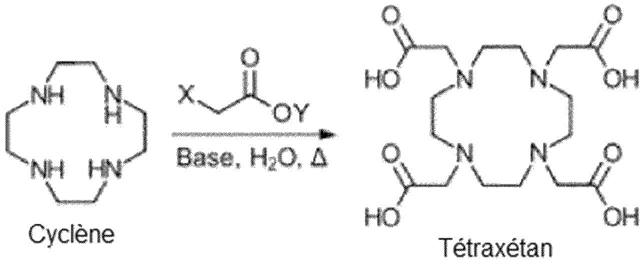 où X est un halogène, Y est choisi parmi l'hydrogène ou un élément alcalin, la base est choisie parmi l'hydroxyde de potassium, de sodium ou de lithium, le pH est maintenu entre 7 et 8,5 et la température de réaction est maintenue entre 70 et 100ºC, et cristallisation du tétraxétan obtenu par abaissement du pH en dessous de 3 pour obtenir un tétraxétan brut;
(b) purification du tétraxétan brut cristallisé obtenu à l'étape précédente, comprenant au moins une électrodialyse;
(c) isolement du produit obtenu à l'étape précédente par séchage par pulvérisation.

2. Procédé selon la revendication 1, dans lequel X est le chlore et/ou Y est le sodium.

3. Procédé selon l'une quelconque des revendications 1 ou 2, dans lequel la base est l'hydroxyde de sodium.

4. Procédé selon l'une quelconque des revendications 1 à 3, dans lequel le pH de la réaction est maintenu à 8 et/ou la température est maintenue à 80ºC.

5. Procédé selon l'une quelconque des revendications 1 à 4, dans lequel le pH dans la cristallisation de l'étape (a) est inférieur à 2, de préférence inférieur à 1.

6. Procédé selon l'une quelconque des revendications 1 à 5, dans lequel des membranes cationiques, anioniques, bipolaires ou des combinaisons de celles-ci sont utilisées en électrodialyse.

7. Procédé selon l'une quelconque des revendications 1 à 6, dans lequel, dans l'étape (b) de purification, deux électrodialyses consécutives sont réalisées.

8. Procédé selon la revendication 7, dans lequel la première des deux électrodialyses est réalisée en utilisant:
- une combinaison de membranes cationiques, anioniques et bipolaires ou
- une combinaison de membranes cationiques et anioniques, où de préférence les membranes anioniques sont monosélectives.

9. Procédé selon l'une quelconque des revendications 7 ou 8, dans lequel la deuxième électrodialyse est réalisée en utilisant:
- une combinaison de membranes cationiques, anioniques et bipolaires ou
- une combinaison de membranes cationiques et bipolaires.

10. Procédé selon l'une quelconque des revendications 7 à 9, dans lequel, dans les deux électrodialyses, le pH est maintenu entre 2 et 6.

11. Procédé selon l'une quelconque des revendications 1 à 6, dans lequel, dans l'étape (b) de purification, une nanofiltration à volume constant est réalisée avant l'électrodialyse.

12. Procédé selon la revendication 11, dans lequel l'électrodialyse est réalisée en utilisant :
- une combinaison de membranes cationiques, anioniques et bipolaires ou
- une combinaison de membranes cationiques et anioniques, où de préférence les membranes anioniques sont monosélectives ou
- une combinaison de membranes cationiques et bipolaires.

13. Procédé selon l'une quelconque des revendications 11 ou 12, dans lequel le pH pendant la nanofiltration est maintenu entre 2 et 8, de préférence entre 3 et 5, plus préférablement à 4.

14. Procédé selon l'une quelconque des revendications 11 à 13, dans lequel le pH pendant l'électrodialyse est maintenu entre 2 et 5, de préférence à 4.

15. Procédé d'obtention de la méglumine de gadotérate comprenant les étapes du procédé selon les revendications 1 à 14 et les étapes supplémentaires suivantes:
(d) réaction du produit obtenu à l'étape (c) avec Gd₂O₃ et la méglumine
(e) isolement du produit obtenu à l'étape (d) par séchage par pulvérisation, de préférence la température de l'air d'entrée dans le système de séchage par pulvérisation étant de 160-200ºC, de préférence de 170-190ºC, plus préférablement de 175-185ºC, encore plus préférablement de 180ºC et/ou la température de l'air de sortie étant de 90-120ºC, de préférence de 105-115ºC, plus préférablement de 110ºC.
